(19) Europäisches Patentamt
European Patent Office
Office européen des brevets

(11) EP 2 832 319 A1

(12) **EUROPEAN PATENT APPLICATION**

(43) Date of publication:
04.02.2015 Bulletin 2015/06

(51) Int Cl.:
*A61F 2/24* (2006.01)     *A61F 2/848* (2013.01)

(21) Application number: 13178818.4

(22) Date of filing: 31.07.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(71) Applicant: **Universitätsklinikum Jena**
**07743 Jena (DE)**

(72) Inventors:
• **Figulla, Hans Reiner, Prof. Dr. med.**
**07749 Jena (DE)**
• **Lauten, Alexander, Dr. med.**
**07749 Jena (DE)**

(74) Representative: **Schulz, Ben Jesko**
**Schulz Junghans**
**Patentanwälte PartGmbB**
**Großbeerenstraße 71**
**10963 Berlin (DE)**

(54) **Implant for bundling a plurality of tendons, particularly chordae tendineae**

(57) The present invention relates to an implant for bundling a plurality of tendons (6), particularly chordae tendineae, wherein a three-dimensionally curved element (1), revolves around a central axis (2) at a radius (3) with respect to the central axis (2) and wherein said radius (3) is larger at a first portion (4) of said curved element (1) than at a second portion (5) of said curved element (1) and wherein said curved element (1) is designed to encompass said plurality of tendons (6) and to bundle them, causing an effective shortening of the tendons (6) along the central axis (2) of the curved element (1).

Figure 10

**Description**

The present invention relates to an implant for bundling a plurality of tendons, particularly chordae tendineae (also known tendinous chords or heart strings) of a human or animal heart.

A closing inability of either the mitral valve or tricuspid valve is one of the main valvular heart diseases during advanced age. In the majority of all cases (>90%) an enlargement of the right or left atrium causes a divergence of the papillary muscles and papillary tendons, which in turn leads to a "relative" valve insufficiency.

The mitral valve has two cusps and lies between the left atrium and the left ventricle, whereas the tricuspid valve has usually three cusps and lies between the right atrium and the right ventricle.

The mitral valve and the tricuspid valve are known collectively as the atrioventricular valves as they lie between the atria and the ventricles of the heart and control the flow of blood.

The function of said valves is to open during diastole and to close at the end of atrial contraction to prevent back flow of blood into the respective atrium.

The cusps of said valves are prevented from prolapsing into the atrium by the action of tendons attached to the valve, the so-called chordae tendineae. These tendons connect the papillary muscles with the valve and thus transmit the pulling force of said muscles to the valves for every heartbeat. This constant strain will eventually wear these tendons and the surrounding tissue out, so that the tissue and the tendons might be overstretched or become too long so that the shutting of the valve is incomplete, which leads to gradually increasing complications.

It is known that it is possible to use artificial tendons for correcting insufficiencies which arise due to worn out tendons or for replacing ripped tendons.

It is furthermore known that such valve surgeries are performed mainly not minimal invasively, but on the open heart, as the surgeon has a better view on the cusps. This however bears greater risks for the patient due to the complex and extensive nature of such kind of surgeries.

Therefore, the problem underlying the present invention therefore is to provide an implant allowing for a proper closing of the atrioventricular valve, which can be particularly used within the framework of a minimal invasive surgery.

This problem is solved by an implant having the features of claim 1. Preferred embodiments are stated in the sub claims.

According to claim 1, an implant for bundling a plurality of tendons, particularly chordae tendineae of humans or animals, comprises a three-dimensionally curved element, wherein said curved element is arranged circumferentially around an (e.g. virtual) central axis and wherein the radius of said curved element is larger at a first portion of said curved element than at a second portion of said curved element, wherein said curved element is designed to encompass said plurality of tendons and to bundle them in the radial direction, causing an effective shortening of the tendons along the direction of the central axis of the curved element.

In other words, the present invention particularly discloses an implant for adapting the papillary muscles, tendons and cusps, by effectively shortening the tendons along their direction of extent and thus particularly correcting a shutting insufficiency of the valve. Said shortening is particularly facilitated by gathering said tendons with the curved element such that said tendons are deflected along a radial direction (i.e. mostly along the orthogonal direction of the direction of extent of the tendons) as the curved element poses a radial constraint, which varies along the central axis of said curved element.

In a preferred embodiment of the invention, the curved element spirals up - particularly continuously as wells as particularly monotonically - along the central axis (or revolves/extends several times around said central axis), so that the curved element describes a three-dimensional spiral or helix.

According to a preferred embodiment of the invention, the radius of the curved element decreases continuously and particularly monotonically from the first portion of the curved element towards the second portion of the curved element.

According to a preferred embodiment of the present invention, the curved element spirals up the central axis at a defined slope, wherein said slope is particularly constant. This corresponds to a constant pitch. It is however also possible to use a curved element which comprises varying slopes.

The slope of such a curved element is particularly chosen such that the curved element comprises preferably e.g. less than one revolution per mm along the central axis.

According to a further preferred embodiment of the present invention, said curved element comprises a predefined handedness, which is either right- or left-handed, defining a corresponding coiling direction along which the curved element extends around or spirals up the central axis from the first portion (or end) towards the second portion (or end).

If, for example, an observer with the line of sight along the central axis of the curved element, looks from the first portion (larger radius) towards the second portion (smaller radius) and said curved element spirals away from the observer (towards the second portion) in a clockwise manner, then said curved element is called right-handed (and its coiling direction runs clockwise). If, however the curved element spirals away in an anti-clockwise manner (towards the second portion), it is called left-handed, and its coiling direction runs anti-clockwise.

In a preferred embodiment of the invention said curved element comprises a first end and a second end, wherein said radius of the curved element is larger at the first end than at the second end.

In a preferred embodiment of the present invention said curved element is designed to gather the tendons by turning the curved element around the tendons, particularly by threading the tendons at the first end of the curved element.

Starting particularly at the first end (where e.g. the radius of the curved element is largest), said curved element is arranged at least partially around the plurality of tendons by turning said curved element, particularly around its central axis, so as to thread the tendons by means of the first end of the curved element while the central axis of said curved element is aligned with the direction of extent of the plurality of tendons. With each turn of the curved element, the tendons encompassed by the curved element will experience the tighter radial constraint due to the diminishing radius of the curved element, and thus will be bundled increasingly. The degree of bundling is therefore adjustable by the amount of turns used for bundling. The maximum bundling capability is inter alia defined by the minimum radius of the curved element.

According to a preferred embodiment of the present invention said curved element comprises a protrusion or several protrusions projecting from said curved element, wherein particularly said protrusions comprise a free end portion, wherein said end portions of said protrusions each extend along a direction that has at least a component in the coiling direction. Particularly, said at least one protrusion is designed to inhibit a motion of the curved element along said coiling direction upon turning of the curved element counter to the coiling direction or when said plurality of tendons is finally encompassed by said curved element as intended.

Said at least one protrusion or said plurality of protrusions particularly ensures that after surgery the implant does not move out of place such that the bundled tendons do not become loose again.

The protrusions may be distributed along the curved element in an equidistant manner.

In a preferred embodiment of the present invention said at least one protrusion (or said plurality of protrusions) may be formed as a barb and/or may comprise a barb. Such a barb may be designed to puncture a tendon to be bundled in order to prevent the curved element from motions in the coiling direction.

Further, such a barb may also be formed such that it encompasses a tendon and thereby catches it, particularly without penetrating the tendon or other tissue.

The protrusions may be integrally formed with or connected to the curved element by e.g. welding or gluing or even other suitable methods.

According to a preferred embodiment of the invention, the curved element spans a fictitious envelope, which is particularly rotationally symmetric with respect to the central axis, and wherein said envelope comprises a surface line that is particularly convex, concave or straight, wherein a straight surface line particularly corresponds to a curved element forming a conic helix.

A concave surface line is particularly achieved when the radius of the curved element decreases with each revolution, e.g. hyperbolically, whereas a convex surface line is achieved by a parabolic decrease of the radius with each revolution of the curved element.

In a preferred embodiment of the present invention, the implant according to the invention is made of or comprises a metal or a metal alloy, particularly comprising nickel and titanium, wherein particularly nickel and titanium are comprised in an equal molar ratio in said alloy, i.e. Nitinol.

Preferred materials are for example those materials which are suitable for surgery and other implants. The curved element is particularly an elastic but dimensionally stable wire. An example for an elastic, but dimensionally stable material is e.g. Nitinol (see also above).

According to a preferred embodiment of the present invention, the curved element comprises a length between 5 mm and 50 mm along the direction of the central axis, particularly 5 mm, preferably 10 mm, preferably 15 mm, preferably 20 mm, preferably 25 mm, preferably 30 mm, preferably 35 mm, preferably 40 mm, preferably 45 mm, or preferably 50 mm. For example, a conic helix-shaped curved element will measure between 5 mm - 50 mm from its base to its apex along the central axis.

In a preferred embodiment of the invention the radius of said curved element has a maximum value, which is preferably less than or equal to 23 mm at the first end of the curved element. Preferably, said radius particularly measures 50% of said maximum value, particularly 11.5 mm, at the second end of the curved element. Further, said radius preferably decreases linearly with each revolution around said central axis, so that the curved element forms a conic helix.

It is however noted that it is not explicitly excluded that said radius decreases following a different function or course such as e.g. exponentially, logarithmic, parabolic or hyperbolic.

Further features and advantages of the invention shall be described by means of detailed descriptions of embodiments of the present invention with reference to the Figures, wherein

Fig. 1        shows a schematic plan view onto an apex of an implant according to the invention;

Fig. 2A        shows a perspective view of a curved element according to the invention in the form of a left-handed conic helix;

Fig. 2B        shows a plan view onto the apex of the curved element shown in Fig. 2A;

Fig. 3A        shows a perspective view of a curved element according to the invention in the form of a right-handed conic helix;

Fig. 3B        shows a plan view onto the apex of the curved element shown in Fig. 3A;

Fig. 4A        shows a perspective view of a curved element according to the invention formed as a left-handed helix comprising a convex surface line;

Fig. 4B        shows a plan view onto the apex of the curved element of Fig. 4A;

Fig. 5A        shows a perspective view of a curved element according to the invention formed as a left-handed helix comprising a concave surface line;

Fig. 5B        shows a plan view onto the apex of the curved element shown in Fig. 5A;

Fig. 6A        shows a perspective view of a curved element according to the invention formed as a left-handed, elliptical helix comprising a straight surface line;

Fig. 6B        shows a plan view onto the apex of the curved element shown in Fig. 6A;

Fig. 7A        shows a perspective view of a curved element according to the invention formed as a left-handed helix comprising a concave surface line, particularly a logarithmic helix;

Fig. 7B        shows a plan view onto the apex of the curved element shown in Fig. 7A;

Fig. 8A        shows a perspective view of a curved element according to the invention formed as a left- handed, helix comprising a variable pitch;

Fig. 8B        shows a plan view onto the apex of the curved element shown in Fig. 8A;

Fig. 9         shows a curved element according to the invention formed as a left-handed hourglass; and

Fig. 10A-B     shows the implant according to the invention encompassing tendons for effectively shortening said tendons.

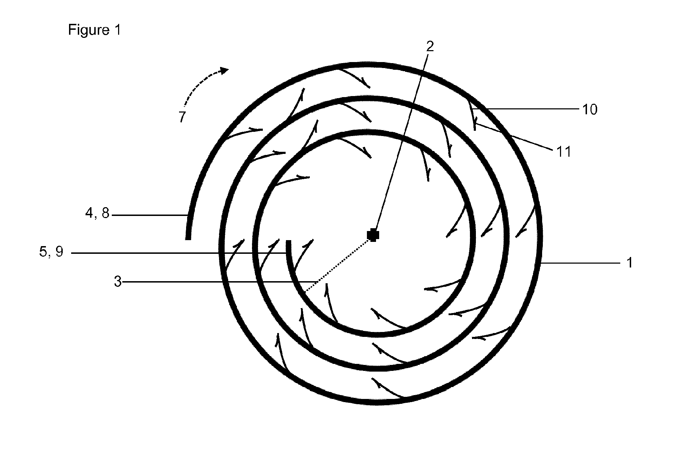

Figure 1 shows a schematic view of an implant according to the invention, wherein Fig. 1 shows a plan view of a left-handed curved element 1 that may have a conical shape. Here, the curved element 1 extends towards the viewer describing a spiral, which spirals up in a coiling direction 7 around a fictitious central axis 2 with a decreasing radius 3. Several protrusions 10 protrude from the curved element 1. These protrusions 10 each have a free end portion 11, which points in a direction that comprises a component in the coiling direction 7. The end portions 11 of said protrusions 10 serve for barbing tendons 6 encompassed by the curved element 1. Once tendons 6 (extending at least piecewise along the central axis 2) are encompassed by the curved element 1, turning the latter in the coiling direction 7 is prevented by the protrusions 10. Thus, said protrusions 10 provide means for a final anchoring of the implant, which prevent an autonomous turning of the implant once in place. Such an implant is typically inserted into the heart by a catheter via an access through a vein, artery or the top of the ventriculum. The distance between the protrusions 10 does not have to be equidistant.

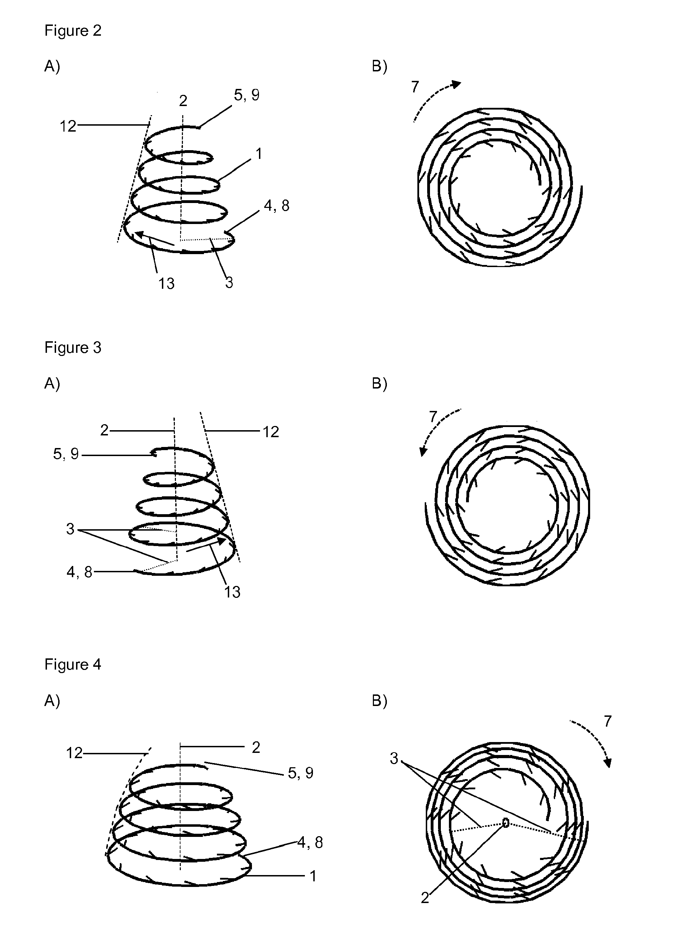

Figure 2 to Figure 9 show various embodiments of the curved element 1 according to the invention. Such shapes and sizes of curved elements 1 can be parameterised with the following equation (Eq.1) for a Cartesian coordinate system.

$$\begin{pmatrix} x(t) \\ y(t) \\ z(t) \end{pmatrix} = \begin{pmatrix} a_x \cdot r(t) \cdot \cos(2\pi n \cdot t) \\ a_y \cdot r(t) \cdot \sin(2\pi n \cdot t) \\ h_0 \cdot h(t) \end{pmatrix}, \text{ with} \qquad\qquad (Eq.1)$$

$t \in [0,1]$; as a parameter taking values between 0 and 1.

$r(t) = r_0 + b \cdot f(t)$ as a radius function

$f(t) = t^\alpha, e^{\beta t}$ ; as a radial course-describing function

$h(t) = t^\gamma, e^{\delta t}$; as a height function describing.

A basic parameter set $P$ is given by the following value set of parameters:

$\alpha = \gamma = a_x = a_y = 1$; $n=4$; $r_0 = b = 10$ $mm$; $h_0 = 20$ $mm$; $f(t) = h(t) = $ t

By varying one or more of these parameters in $P$ different shaped helices (curved elements) will result. The following table shows various examples of helices, and their specific parameter set. The shapes listed in table 1 are exemplary displayed in Figures 3-9.

Table 1:

| Resulting shape | Parameter set |
|---|---|
| conic helix | $P$ |
| conic helix of opposite handedness | $P$ and $a_y = -1$ |
| elliptical, conic helix | $P$ and $a_x \neq a_y$ |
| Convex envelope helix | $P$ and $\alpha < 1$ |
| Concave envelope helix | $P$ and $\alpha > 1$ |
| Variable pitch helix | $P$ and $\gamma \neq 1 \rightarrow h(t) = t^\gamma$ or $\delta \neq 1 \rightarrow h(t) = e^{\delta t}$ |
| Logarithmic helix | $P$ and $\beta \neq 0 \rightarrow f(t) = e^{\beta t}$ |

Figure 2 shows schematic views of a curved element 1 formed as a left-handed conic helix. Figure 2A shows a perspective view of said curved element 1. The envelope of such a helix comprises a straight surface line 12, which is an indicator for a conic helix. The helix features a constant pitch. The radius 3 is decreasing from the first end 8 towards the second end 9 while the curved element 1 spirals up around the central axis 2. Further, Fig. 2B shows a plan view (view of the curved element 1 from its second end 9 (apex) towards the first end 8). The helix turns clockwise towards the observer in a coiling direction 7. The curved element comprises protrusions 10 for anchoring the implant according to the invention, which protrusions 10 are pointing in a direction that comprises a component in the coiling direction 7.

Figure 3 shows schematic views of a curved element 1 formed as a right-handed conic helix. Fig. 3A shows a perspective view of said curved element 1. The envelope of such a helix comprises a straight surface line 12, which is an indicator for a conic helix. The helix features a constant pitch. The radius 3 is decreasing from the first end 8 towards the second end 9 while the curved element spirals 1 up around the central axis 2. Figure 3B shows a plan view onto the apex 5 of the curved element 1 (see above). Correspondingly, the helix turns anti-clockwise towards the observer in the coiling direction 7. Again, the protrusions 10 are pointing in a direction that comprises a component in the coiling direction 7 so as to prevent the implant from loosening.

Figure 4 shows schematic views of a curved element 1 formed as a left-handed curved element 1. Figure 4A shows a perspective view of said curved element 1. The envelope of such a helix comprises a convex surface line 12 defining a convex helix. The helix features a constant pitch. Figure 4B shows the plan view (view of the curved element 1 from its second end 9 towards the first end 8). The helix turns clockwise towards the observer in said coiling direction 7.

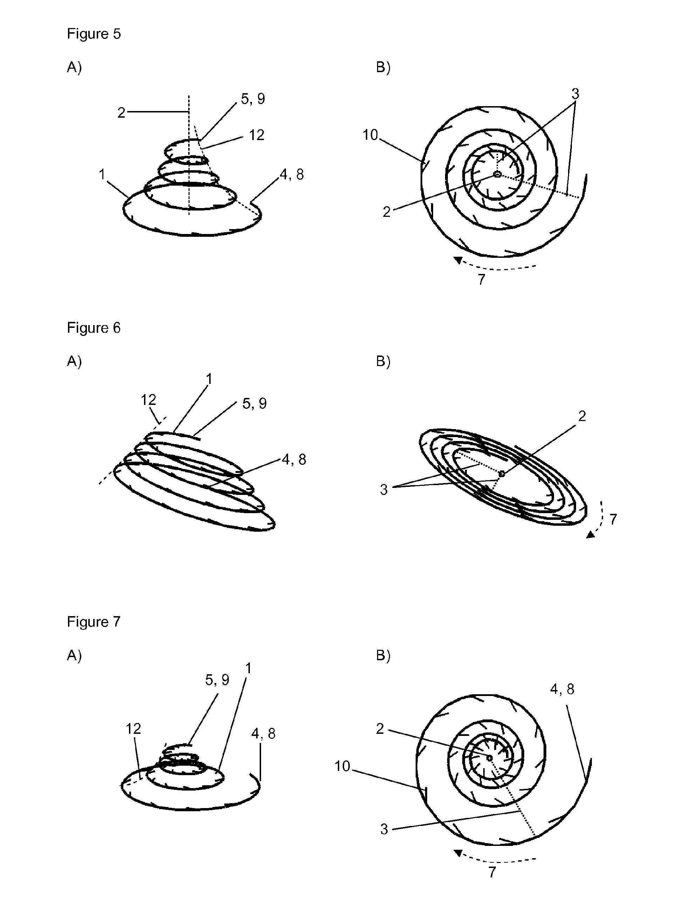

Figure 5 shows schematic views of a curved element 1 formed as a left-handed curved element 1. Figure 6A shows a perspective view of said curved element 1. The envelope of such a helix comprises a concave surface line 12, defining a concave helix. The helix, and thus the curved element 1, features a constant pitch. Figure 6B shows the plan view (view of the curved element 1 from its second end 9 towards the first end 8). The helix turns clockwise towards the observer in the coiling direction 7.

Figure 6 shows schematic views of a curved element 1 formed as a left-handed curved element 1. Figure 6A shows a perspective view of said curved element 1. The envelope of such a helix comprises a straight surface line 12 defining a conic helix. As the curved element 1 is elliptic it is described by an elliptical, conic helix. The helix, and thus the curved element 1, features a constant pitch. Figure 6B shows the plan view (view of the curved element 1 from its second end 9 towards the first end 8). The helix turns clockwise towards the observer in the coiling direction 7.

Figure 7 shows schematic views of a curved element 1 formed as a left-handed curved element 1. Figure 7A shows a perspective view of said curved element 1. The envelope of such a helix comprises a concave surface line 12 defining a concave helix, in this case particularly a logarithmic spiral. Figure 7B shows the plan view (view of the curved element 1 from its second end 9 towards the first end 8). The helix turns clockwise towards the observer in said coiling direction 7.

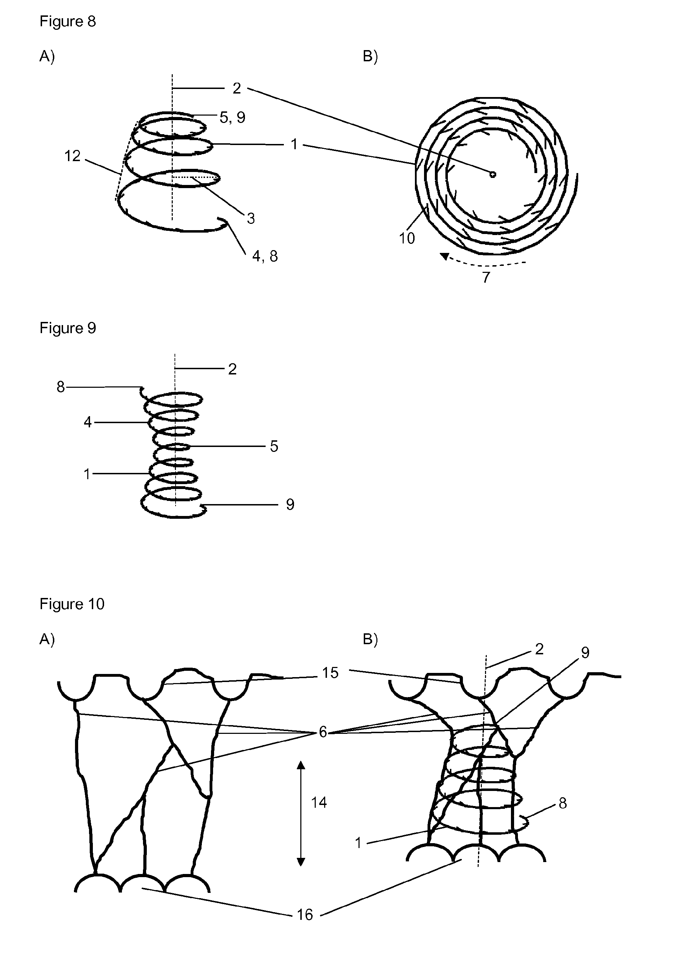

Figure 8 shows schematic views of a curved element formed as a left-handed curved element 1. Figure 8A shows a perspective view of said curved element 1. The helix, and thus the curved element 1, features a variable pitch. In this case, the slope of the curved element 1 is decreasing with decreasing radius 3. Thus, the curved element 1 becomes more dense as the radius 3 decreases providing more protrusions 10 as the radius 3 decreases. Figure 8B shows the plan view (view of the curved element 1 from its second end 9 towards the first end 8). The helix turns clockwise towards the observer in the clockwise coiling direction 7.

Figure 9 shows a perspective view of a curved element 1 formed as a left-handed curved element 1. The curved element 1 spans a virtual envelope that is formed as an hourglass, i.e., it comprises a narrow portion in the middle of the curved element 1. Thus, it comprises a first portion 4 where the radius is larger than at a second portion 5. At the ends 8, 9 the curved element 1 comprises essentially the same radius. The curved element 1 is a helix, with a constant pitch.

Figure 10 shows a schematic drawing of chordae tendineae 6. These tendons 6 connect the muscles 15 and the valves 16 of a human heart. The tendons 6 in Figure 10A are interconnected with each other, but have a main direction of extension 14. In Figure 10B the implant is arranged around the tendons 6 and causes an effective shortening of the tendons 6 along the direction of the central axis 2, by deflection of the tendons 6 in a radial direction and by bundling the tendons 6.

**Claims**

1. Implant for bundling a plurality of tendons (6), particularly chordae tendineae,
**characterized by**
a three-dimensionally curved element (1), wherein said curved element (1) revolves around a central axis (2) at a radius (3) with respect to the central axis (2) and wherein said radius (3) is larger at a first portion (4) of said curved element (1) than at a second portion (5) of said curved element (1) and wherein said curved element (1) is designed to encompass said plurality of tendons (6) and to bundle them, causing an effective shortening of the tendons (6) along the central axis (2) of the curved element (1).

2. Implant according to claim 1, **characterized in that** the curved element (1) spirals up along the central axis (2), so that the curved element (1) forms a three-dimensional spiral.

3. Implant according to claim 1 or 2, **characterized in that** the radius (3) decreases continuously and particularly monotonically from the first portion (4) of the curved element (1) towards the second portion (5) of the curved element (1).

4. Implant according to one of the preceding claims, **characterized in that** the curved element (1) spirals up the central axis (2) at a defined slope (13), wherein said slope (13) is particularly constant.

5. Implant according to one of the preceding claims, **characterized in that** said curved element (1) comprises a predefined handedness, which is either right-handed or left-handed, defining a corresponding coiling direction (7) along which the curved element (1) spirals up along the central axis (2) from the first portion (4) to the second portion (5), respectively.

6. Implant according to one of the preceding claims, **characterized in that** said curved element (1) comprises a first end (8) and a second end (9), wherein said radius (3) is larger at the first end (8) than at the second end (9) of the curved element (1).

7. Implant according to one of the preceding claims, **characterized in that** said curved element (1) is designed to bundle the plurality of tendons (6) by turning the curved element (1) around the tendons (6), particularly by threading the tendons (6) at the first end (8) of the curved element (1).

8. Implant according to one of the claims 5 to 7, **characterized in that** said curved element (1) comprises at least one protrusion (10) protruding from said curved element (1), wherein said at least one protrusion (10) comprises an end portion (11), wherein said end portion (11) of the at least one protrusion points in a direction that has a non-vanishing positive component in said coiling direction (7), wherein said at least one protrusion (10) is designed to inhibit a motion of the curved element (1) along said coiling direction (7) when said plurality of tendons (6) is encompassed by said curved element (1) at least in sections.

9. Implant according to claim 8, **characterized in that** said at least one protrusion (10) forms and/or comprises a barb.

10. Implant according to one of the preceding claims, **characterized in that,** the curved element (1) spans a virtual envelope, which is particularly rotationally symmetric with respect to the central axis (2), and wherein said envelope comprises a surface line (12), and wherein said surface line (12) is particularly convex, concave or straight, wherein particularly a straight surface line (12) corresponds to a curved element (1) in the form of a conic helix.

11. Implant according to one of the preceding claims, **characterized in that** the curved element (1) comprises a metal or a metal alloy, particularly comprising nickel and titanium, wherein particularly nickel and titanium are comprised in an equal molar ratio in said alloy.

12. Implant according to one of the preceding claims, **characterized in that** the curved element (1) comprises a length between 5 mm and 50 mm along the central axis (2).

13. Implant according to one of the claims 6 to 12, **characterized in that** the radius (3) of said curved element (1) has a maximum value, which is less than or equal to 23 mm at the first end (8) of the curved element (1), and wherein particularly said radius (3) measures 50% of said maximum value, particularly 11,5 mm, at the second end (9) of the curved element (1).

14. Implant according to one of the preceding claims, **characterized in that** said radius (3) decreases linearly with each revolution of the curved element (1) around said central axis (2).

Figure 1

Figure 2

A)

B)

Figure 3

A)

B)

Figure 4

A)

B)

Figure 5

A)

B)

Figure 6

A)

B)

Figure 7

A)

B)

Figure 8

A)

B)

Figure 9

Figure 10

A)

B)

Europäisches
Patentamt

European
Patent Office

Office européen
des brevets

## EUROPEAN SEARCH REPORT

Application Number

EP 13 17 8818

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/006352 A1 (YARON IRA [IL]) 3 January 2013 (2013-01-03) * paragraphs [0007] - [0008], [0031] - [0090]; figures 1-15, 18-20B * | 1-14 | INV. A61F2/24 ADD. A61F2/848 |
| X | US 2007/255396 A1 (DOUK NAREAK [US] ET AL) 1 November 2007 (2007-11-01) * paragraphs [0038] - [0043], [0050]; figures 2-8, 15-18 * | 1-7, 10-12,14 | |
| X | EP 2 072 027 A1 (MEDTENTIA LTD OY [FI]) 24 June 2009 (2009-06-24) * paragraphs [0041] - [0049], [0129] - [0138]; figures 14-16, 19 * | 1-7,10, 11,14 | |
| A | WO 2013/028387 A2 (TENDYNE HOLDINGS INC [US]) 28 February 2013 (2013-02-28) * paragraphs [0429] - [0432]; figures 22, 24 * | 1,2,5-7, 11,14 | |

TECHNICAL FIELDS
SEARCHED    (IPC)

A61F

The present search report has been drawn up for all claims

| Place of search | Date of completion of the search | Examiner |
|---|---|---|
| The Hague | 2 January 2014 | Porta, Marcello |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 13 17 8818

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2014

| Patent document cited in search report | Publication date | Patent family member(s) | Publication date |
|---|---|---|---|
| US 2013006352 A1 | 03-01-2013 | US 2013006352 A1<br>WO 2013001339 A2 | 03-01-2013<br>03-01-2013 |
| US 2007255396 A1 | 01-11-2007 | US 2007255396 A1<br>WO 2004112651 A2 | 01-11-2007<br>29-12-2004 |
| EP 2072027 A1 | 24-06-2009 | AU 2008339967 A1<br>CA 2710360 A1<br>CN 102131479 A<br>EP 2072027 A1<br>EP 2222248 A1<br>JP 2011506017 A<br>KR 20100120123 A<br>RU 2010130470 A<br>US 2010331971 A1<br>WO 2009080801 A1 | 02-07-2009<br>02-07-2009<br>20-07-2011<br>24-06-2009<br>01-09-2010<br>03-03-2011<br>12-11-2010<br>27-01-2012<br>30-12-2010<br>02-07-2009 |
| WO 2013028387 A2 | 28-02-2013 | NONE | |

EPO FORM P0459

For more details about this annex : see Official Journal of the European Patent Office, No. 12/82